# EUROPEAN PATENT APPLICATION

(11) **EP 1 666 460 A1**
(43) Date of publication of application: **07.06.2006**
(21) Application number: 04773136.9
(22) Date of filing: 09.09.2004
(51) Int. Cl.: C07D 207/34, A61K 31/40, A61P 9/10, A61P 9/12, A61P 25/00

(54) **PREVENTIVE FOR CEREBRAL STROKE RECURRENCE**

(30) Priority: 11.09.2003 JP 2003319131
(71) Applicant: YAMANOUCHI PHARMACEUTICAL CO., LTD., Tokyo 103-8411 (JP)
(72) Inventor: HAYASHI, Asuka Astellas Pharma Inc., Chuo-ku, Tokyo 103-8411 (JP); SUZUKI, Masanori Astellas Pharma Inc., Chuo-ku, Tokyo 103-8411 (JP); SASAMATA, Masao Astellas Pharma Inc., Chuo-ku, Tokyo 103-8411 (JP)
(74) Representative: Bates, Philip Ian
(86) International application number: PCT/JP2004/013477
(87) International publication number: WO 2005/026117

(57) **Abstract**

The invention provides a novel cerebral stroke recurrence inhibitor, and relates to a simple evaluation method for an cerebral stroke recurrence inhibitor.

According to the simple evaluation method using stroke-prone spontaneously hypertensive (SHR-SP) rats, which has been established by the present inventors, it has been confirmed that atorvastatin is effective for preventing cerebral stroke recurrence, for relieving cerebral stroke recurrence-accompanied neurologic symptoms, and for improving survival rate after onset of the stroke. Accordingly, atorvastatin is useful as an cerebral stroke recurrence inhibitor.

## Description

### TECHNICAL FIELD

The present invention relates to an cerebral stroke recurrence inhibitor, a neurologic symptom reliever in cerebral stroke recurrence or a survival rate improver after cerebral stroke attack, which comprise atorvastatin or its salt as an active ingredient, and to a method for evaluating the effect relating to cerebral stroke recurrence.

### BACKGROUND ART

Cerebral stroke is a generic term for brain hemorrhage caused by breakage of a blood vessel in a brain, and brain infarction caused by blood vessel obstruction by thrombus in a brain, and is referred to as one of the cerebrovascular events.

Cerebral stroke accounts for a large proportion of the cause of death in advanced nations, and it is a disorder having a high risk of recurrence. As a result of a survey in Japan, there is a report saying that the recurrence ratio of cerebral stroke cases with hypertension is 7.5%/year/patient; and the Framingham study in USA gives a report saying that the 5-year cumulative recurrence ratio of brain infarction is 42% in men and 24% in women (Non-Patent Reference 1).

In many cases, the convalescence of cerebral stroke recurrence is worse than that of primary cerebral stroke attack. In many cases, a primary cerebral stroke attack may often leave simple hemiplegia, but the cerebral stroke recurrence would leave complicated and more serious disorders such as systemic paralysis, trunk paralysis, swallowing failure, communication insufficiency, dysuria, dementia, agnosia, and its prevention is a medical and social problem of the great importance.

For preventing cerebral stroke recurrence, the mainstream is pharmacotherapy by administration of an antiplatelet (especially aspirin) or an anticoagulant such as heparin, warfarin potassium, which, however, requires long-term administration of the drug and is problematic in that different drugs must be used depending on the type of the disease and that the dosing may have a side effect of hemorrhage; and more effective drugs are desired.

Atorvastatin has a strong HMG-CoA reductase-inhibiting effect and is widely used all over the world as an excellent cholesterol depressant. In various animal tests or in large-scale clinical tests with it, other various effects of the compound in addition to the cholesterol-depressing effect thereof have been revealed.

Regarding the large-scale clinical tests with atorvastatin relating to cardiovascular events including cerebral stroke, there are some reports showing the results of a test of Anglo-Scandinavian Cardiac Outcomes Trial-Lipid Lowering Arm (ASCOT-LLA) for the purpose of evaluating the primary prevention of a cardiovascular event of target cases with hypertension not having had a coronary heart disease (CHD) in the past but having at least three CHD risk factors (Non-Patent Reference 2), a test of Myocardial Ischemia Reduction with Aggressive Cholesterol Lowering (MIRACL) for the purpose of evaluating the secondary prevention of a cardiovascular event by atorvastatin administration in the acute stage after a coronary event attack (Non-Patent Reference 3), a test of Greek Atorvastatin and Coronary-Heart-Disease Evaluation (GREACE) of comparing the CHD secondary prevention effect of CHD-having cases with an ordinary therapeutical effect (Non-Patent Reference 4).

From the results of these large-scale clinical tests, it has become clear that atorvastatin is effective for preventing a primary cerebral stroke in hypertension cases or in CHD-having cases, or that is, it has a primary preventive effect for cerebral stroke.

At present, large-scale clinical tests are being carried out for evaluating the secondary preventive effect of atorvastatin for cases with TIA or cerebral stroke within 1 to 6 months (Non-Patent Reference 5), but no result has been obtained as yet from them, and the effect of atorvastatin for preventing cerebral stroke recurrence has not as yet been clarified.

On the other hand, in a test with brain infarction model animals, it has been known that atorvastatin is effective for reducing the brain infarction volume when it is administered for 3 days before the brain infarction attack. In addition, it has been confirmed that atorvastatin increases eNOS activity. It is believed that the increase in eNOS activity may contribute to the reduction in a brain infarction volume by preventive administration owing to the bloodstream improving effect of it through vasodilation (Patent Reference 1). However, the correlation between eNOS activity and cerebral stroke recurrence prevention has not as yet become clarified up to now.

Regarding a report of using stroke-prone spontaneously hypertensive rats (SHR-SP) used in the evaluation method of the invention, there is a report of sympathetic control effect of atorvastatin based on the NO synthetase activation capability thereof (Non-Patent Reference 6), but nothing has been investigated therein relating to cerebral stroke.

On the other hand, there is a report using SHR-SP rats relating to serivastatin, a type of an HMG-CoA reductase inhibitor (statin), which says that the serivastatin is effective for prevention of cerebral stroke attack, effective for increasing a survival rate, and effective for reducing a brain infarction volume (Non-Patent Reference 7). However, these are all by administration before attack and nothing is investigated relating to administration after cerebral stroke attack.

In another large-scale clinical test with statin of Scandinavian Simvastatin Survival Study (4S) by administration of simvastatin to high-cholesterol cases having an ischemic heart disease, it has been shown for the first time that statins have a primary prevention effect for cerebral stroke (Non-Patent Reference 8). In a test of MRC/BHF Heart Protection Study by administration to cases of coronary arteritis, those of obstructive arteritis or those of diabetes who have an ordinary LDL-cholesterol level, it has been shown that simvastatin has a primary cerebral stroke prevention effect for those subjects with an normal cholesterol level (Non-Patent Reference 9). Further, in Prospective Pravastatin Pooling (PPP) Project, the cerebral stroke-preventing effects in the large-scale clinical tests heretofore carried out are summarized, and it has been shown that pravastatin is effective for preventing non-hemorrhage cerebral stroke attack (for primary prevention) irrespective of the lipid level of the subjects, but there is no difference in hemorrhage-type stroke nor indefinite-type stroke (Non-Patent Reference 10) .

On the other hand, in a test of Long-term Intervention with Pravastatin in Ischemic Disease (LIPID) by administration of pravastatin to hypercholesterolemia cases having a cardiac infarction attack or an unstable angina attack within 3 to 36 months (Non-Patent reference 11), or in a test of Cholesterol and Recurrent Events (CARE) for the purpose of evaluating the relationship between cerebral stroke prevention after cardiac infarction and cholesterol lowering effect (Non-Patent Reference 12), it has been shown that pravastatin is effective for preventing the risk of recurrence of cerebral stroke or transient ischemic attack (TIA).

However, the cerebral stroke recurrence prevention effect is for cases with ischemic heart disease, and heretofore there is found no report relating to the inhibitory effect for cases not having such a underlying disease.

When a case with ischemic heart disease has a vascular accident, it is often recurrence of heart disease, and this presents a problem in point of the reasonableness in discussing the recurrence prevention effect for cerebral stroke attack-having cases with no ischemic heart disease from the previous reports heretofore disclosed in the art (Non-Patent Reference 16).

The matter that the presence or absence of ischemic heart disease may have some influence on cerebral stroke attack may be presumed from the following facts. Specifically, in a test of West of Scotland Coronary Prevention (WOSCOP) for the purpose of evaluating the primary prevention of a coronary heart disease (CHD), the primary cerebral stroke prevention rate is low, different from the data in the previous reports. Regarding the reason, Blauw et al. consider that the WOSCOP test is directed to the cases with no ischemic heart disease different from the LIPID test and the CARE test (Non-Patent Reference 13).

In a test of PROspective Study of Pravastatin in the Elderly at Risk (PROSPER) directed to cardiovascular disorder or cerebral stroke high-risk elderly cases of 70 years or more by administration of pravastatin thereto, it is reported that pravastatin reduced the risk of TIA recurrence but was not ineffective for cerebral stroke recurrence prevention (Non-Patent Reference 14).

As in the above-mentioned reports, it is reported that statins are ineffective for cerebral stroke recurrence prevention for cases not having ischemic heart disease, and the preventive effect of atorvastatin is neither disclosed nor suggested. Regarding the cerebral stroke recurrence prevention effect of statins for hypertensive cases, there has been no reports up to now.

It is considered that the improvement in the factor of primary prevention of cerebral stroke does not always correspond to the factor of recurrence prevention (Non-Patent Reference 1). The risk factors that have heretofore been clarified for cerebral stroke recurrence are only diabetes and atrial fibrillation, and a mere suggestion has been given at present indicating that the cerebral stroke recurrence is complicatedly influenced by a large number of various factors relating to it. Anyhow, the mechanism of cerebral stroke recurrence is not as yet clarified (Non-Patent Reference 15).

In the above-mentioned LIPID test (Non-Patent Reference 11), it is shown that there is no difference between pravastatin administration and placebo administration in the survival rate and the disorder condition after cerebral stroke attack.

Accordingly, there is found no report that may suggest the effect of statins for improving the survival rate and the disorder condition after cerebral stroke attack.

[Non-Patent Reference 1] EBM Journal (2003) 4(1), 64-69, [Non-Patent Reference 2] Lancet (2003) 361, 1149-1158, [Non-Patent Reference 3] Circulation (2002) 106, 1690-1695,
[Non-Patent Reference 4] Current Medical Research and Opinion (2002) 18/4 (220-228),
[Non-Patent Reference 5] Cerebrovascular Diseases (2003) 16, 389-395,
[Non-Patent Reference 6] Journal of Hypertension (2003), Vol. 21, No. 2, pp. 379-386,
[Non-Patent Reference 7] Stroke (2003), 34, 157-163,
[Non-Patent Reference 8] Lancet (1994) 344, 1383-1389, [Non-Patent Reference 9] Lancet (2002) 360, 7-22
[Non-Patent Reference 10]Circulation (2001) 103, 387-392, [Non-Patent Reference 11] New England Journal of Medicine 2000; 343(5): 317-326,
[Non-Patent Reference 12]Circulation (1999) 99, 216-223, [Non-Patent Reference 13]Stroke (1997) 28, 946-950,
[Non-Patent Reference 14]Lancet (2002) 360, 1623-1630, [Non-Patent Reference 15]Stroke (2003) 34, 1457-1463, [Non-Patent Reference 16] *Igaku no ayumi* (Progress of Medicine) (2003), 204(6), 424-428,
[Patent Reference 1] JP-A-2003-73272.

### DISCLOSURE OF THE INVENTION

An object of the invention is to provide a novel cerebral stroke recurrence inhibitor, cerebral stroke recurrence-accompanied neurologic symptom reliever or survival rate improver.

The present inventors have extensively studied so as to attain the above-mentioned object, and, as a result, have established a novel and simple test method with animal model of reproducing cerebral stroke recurrence, and using it, we have confirmed that atorvastatin is effective for preventing cerebral stroke recurrence, effective for relieving cerebral stroke recurrence-accompanied neurologic symptoms and effective for improving the survival rate after cerebral stroke attack, and have completed the present invention including the following:
(1) An cerebral stroke recurrence inhibitor comprising atorvastatin or a pharmaceutically acceptable salt thereof as an active ingredient.
(2) The cerebral stroke recurrence inhibitor of above (1), which is for mammals not having ischemic heart disease as a underlying disease thereof.
(3) The cerebral stroke recurrence inhibitor of above (1) or (2), which is for hypertensive mammals.
(4) An cerebral stroke recurrence-accompanied neurologic symptom reliever comprising atorvastatin or a pharmaceutically acceptable salt thereof as an active ingredient.
(5) A survival rate after onset of the stroke improver comprising atorvastatin or a pharmaceutically acceptable salt thereof as an active ingredient.
(6) A method of preventing cerebral stroke recurrence, which comprises administering an cerebral stroke recurrence-preventive amount of atorvastatin or a pharmaceutically acceptable salt thereof to a mammal.
(7) The method of above (6), wherein an cerebral stroke recurrence-preventive amount of atorvastatin or a pharmaceutically acceptable salt thereof is administered to a mammal not having ischemic heart disease.
(8) The method of above (6) or (7), wherein an cerebral stroke recurrence-preventive amount of atorvastatin or a pharmaceutically acceptable salt thereof is administered to a hypertensive mammal.
(9) A method of relieving cerebral stroke recurrence-accompanied neurologic symptom, which comprises administering an effective amount of atorvastatin or a pharmaceutically acceptable salt thereof to a mammal.
(10) A method of improving the survival rate after cerebral stroke attack, which comprises administering an effective amount of atorvastatin or a pharmaceutically acceptable salt thereof to a mammal.
(11) An cerebral stroke recurrence inhibitor comprising a compound selected from pitavastatin and rosuvastatin or a pharmaceutically acceptable salt thereof as an active ingredient.
(12) The cerebral stroke recurrence inhibitor of above (11), which is for a hypertensive mammal.
(13) A recurred cerebral stroke-related neurologic symptom reliever comprising a compound selected from pitavastatin and rosuvastatin or a pharmaceutically acceptable salt thereof as an active ingredient.
(14) A survival rate after onset of the stroke improver comprising a compound selected from pitavastatin and rosuvastatin or a pharmaceutically acceptable salt thereof as an active ingredient.
   The invention also relates to a method of evaluating the cerebral stroke recurrence inhibitory effect of a compound, the cerebral stroke recurrence-accompanied neurologic symptom-relieving effect thereof or the survival rate after onset of the stroke-improving effect thereof. Concretely, it relates to the following:
(15) A method of evaluating the cerebral stroke recurrence inhibitory effect of a test substance, the cerebral stroke recurrence-accompanied neurologic symptom-relieving effect thereof or the survival rate after onset of the stroke-improving effect thereof, which comprises the following steps:
   (a) observing the body weight change and/or the neurologic symptom of a stroke-prone spontaneously hypertensive rat (SHR-SP) before administration of a test substance thereto,
   (b) selecting the rat that has shown abnormal body weight change and/or neurologic symptom during the observation in step (a),
   (c) administering a test substance to the rat selected in step (b) and observing them for a predetermined period of time.
(16) A method of evaluating the cerebral stroke recurrence inhibitory effect of a test substance, which comprises administering a test substance to a stroke-prone spontaneously hypertensive rat that has had an cerebral stroke attack, followed by checking the rat for the body weight change and/or the neurologic symptom change thereof for the index of the effect of the test substance.
(17) A method of evaluating the cerebral stroke recurrence inhibitory effect of a test substance, which comprises administering a test substance to a stroke-prone spontaneously hypertensive rat that has had an cerebral stroke attack, followed by scoring the degree of the body weight change of the rat.
(18) A method of evaluating the cerebral stroke recurrence-accompanied neurologic symptom-relieving effect of a test substance, which comprises administering a test substance to a stroke-prone spontaneously hypertensive rat that has had an cerebral stroke attack.
(19) A method of evaluating the cerebral stroke recurrence-accompanied neurologic symptom-relieving effect of a test substance, which comprises administering a test substance to a stroke-prone spontaneously hypertensive rat that has had an cerebral stroke attack, followed by scoring the degree of the neurologic symptom of the rat.
(20) A method of evaluating the survival rate after onset of the stroke-improving effect of a test substance, which comprises administering a test substance to a stroke-prone spontaneously hypertensive rat that has had an cerebral stroke attack.

### BRIEF DESCRIPTION OF THE DRAWINGS

Fig. 1 is graphs showing the cerebral stroke recurrence scores with atorvastatin in 7 days and 14 days after cerebral stroke attack.
Fig. 2 is graphs showing the neurologic symptom scores with atorvastatin in 0, 7 and 14 days after cerebral stroke attack.
Fig. 3 is a graph showing the survival rate change with time by atorvastatin administration after cerebral stroke attack.

### BEST MODE FOR CARRYING OUT THE INVENTION

The invention is further described hereinunder.

"Atorvastatin" means (-)-(3R,5R)-7-[2-(4-fluorophenyl)-5-isopropyl-3-phenyl-4-phenylcarbamoyl-1H-pyrrol-1-yl]-3,5-dihydroxyheptanoic acid.

"Pharmaceutically-acceptable salt" is concretely a lithium, calcium, magnesium, aluminum, ferrous or ferric salt, and is preferably a calcium salt.

More preferably, it is atorvastatin calcium hydrate, or that is, (-)-monocalcium bis{(3R,5R)-7-[2-(4-fluorophenyl)-5-isopropyl-3-phenyl-4-phenylcarbamoyl-1H-pyrrol-1-yll-3,5-dihydroxyheptanoatel trihydrate, and a preparation containing the substance as a single active ingredient is commercially sold as Lipitol (R) .

Atorvastatin calcium hydrate includes an amorphous form (W097/03960) and a polymorphic form (W097/03959, WO97/03958, etc.), and their mixtures, as well as isolated single crystal forms (Form I, II, III, IV, or V to VII, etc.) or amorphous forms thereof are employable for the applications of the invention.

Pitavastatin and rosuvastatin are strong HMG-CoA reductase inhibitors.

For pitavastatin or a pharmaceutically acceptable salt thereof, preferred is pitavastatin calcium, or that is, (+)-monocalcium bis{(3R,5S,6E)-7-[2-cyclopropyl-4-(4-fluorophenyl)-3-quinolyl]-3,5-dihydroxy-6-heptanoate}.

For rosuvastatin or a pharmaceutically acceptable salt thereof, preferred is rosuvastatin calcium, or that is, monocalcium bis[(E)-7-[4-(4-fluorophenyl)-6-isopropyl-2-[methyl(methylsulfonyl)amino]pyrimidin-5-yl](3R,5S)-3,5-dihydroxyhept-6-enoate].

"Cerebral stroke" is grouped into hemorrhagic and non-hemorrhagic types, and the hemorrhagic type includes brain hemorrhage, subarachnoid hemorrhage, intracranial hemorrhage accompanied by brain arterial malformation; and the non-hemorrhagic type includes brain infarction. In particular, cerebral stroke means brain hemorrhage and/or brain infarction.

"Brain infarction" is grouped into a thrombotic type (cerebral thrombosis), an embolic type (cerebral embolus) and a hematodynamic type, depending on the origin of thrombi to it. In point of its clinicopathological classification, brain infarction may be grouped into atherothrombotic brain infarction, cardiogenic brain infarction, lacunar infarction, and others.

"Cerebral stroke recurrence inhibition" means that for preventing mammals that had an cerebral stroke attack in the past (mammals with past cerebral stroke attack), preferably humans that had it (cases with past cerebral stroke attack) from again having an cerebral stroke attack, or means that for relieving the recurred cerebral stroke. Preferably, it means that human cases once having had a primary cerebral stroke attack in the past are prevented from again having a next cerebral stroke attack within 3 years, more preferably within 1 year, most preferably within 6 months (in the acute stage). Even more preferably, it means that human cases who have had a primary cerebral stroke attack for the first time are prevented from again having a next cerebral stroke attack.

"Ischemic heart disease" may be referred to as coronary heart disease (CHD), and means cardiac infarction and stenocardia (preferably unstable angina).

"Hypertension" means that the blood pressure of a mammal is higher over the normal range of the species thereof. Regarding humans, it means 1) those having a systolic pressure of 160 mmHg or higher or a diastolic pressure of 100 mmHg or higher or having both the two; or 2) hypertensive cases having a systolic pressure of 140 mmHg or higher or a diastolic pressure of 90 mmHg or higher or having both the two, and having medical treatment.

"Neurologic symptom" means a symptom of an injured nervous function that is under control by the site of a brain having been injured by cerebral stroke, concretely including dyskinesia, allophasis, sensory disturbance, ophthalmologic failure and the like.

"Neurologic symptom relieving" means that the condition of the neurologic symptom mentioned above is relieved or improved after drug administration, as compared with that before drug administration. Concretely, the degree of paralysis or activity of animals or human cases is scored depending on the level of the severity of the symptoms, and when the score is lowered after drug administration than before drug administration, then it is meant by neurologic symptom relieving. Preferably, the score is significantly lowered after drug administration than before drug administration.

"Dyskinesia" means that voluntary movement is difficult or impossible, or could not be attained smoothly owing to the brain injury by cerebral stroke, including motor paralysis and ataxia. In particular, it means motor paralysis.

"Motor paralysis" means that voluntary movement is difficult or impossible owing to the centrostaltic injury by cerebral stroke. Motor paralysis includes hemiplegia, paraplegia, tetraplegia, and monoplegia with spastic or flaccid paralysis.

"Ataxia" means that voluntary movement could not be attained smoothly owing to intermuscular unbalance or incoordination even though the cases have neither muscular weakness nor paralysis.

"Consciousness disorder" means essentially the reduction of a consciousness level and means that the cases could not correctly recognize any external stimulation and could not react to it on the basis of their recognized results. Consciousness disorder includes a consciousness lucidity change such as syncope, lethargy, stupor, hemistupor, coma; and a consciousness content change such as delirium, twilight state.

"Allophasis" includes dysarthria, aphasia.

"Dysarthria" is caused by the injury to the articulation-relating muscular system or the nervous system to govern it, and it is generally expressed as "cannot speak clearly or cannot articulate" or "tongue-tied". Dysarthria includes paralytic, cerebellar, and extrapyramidal disorders, and the injury by cerebral stroke includes pyramidal and cranial nervous disorders.

"Aphasia" indicates an injured condition in such that words could not speak and understand correctly owing to the injury to the speech region of cerebrum, and this may occur irrespective of dyskinesia in articulation organs. In almost all cases, it is caused by the injury to left cerebrum. Aphasia includes Broca aphasia, Wernicke aphasia, complete aphasia, conductive aphasia.

Sense includes superficial sense such as taction, pressure sense, temperature sense; deep sensibility such as localization, pallesthesia; and composite sensibility such as two-point differentiation, on-skin writing sensibility.

"Sensory disturbance" means that these senses could not be correctly recognized owing to the brain disorders, and depending on its degree, it is grouped into sensibility loss (anesthesia), sense torpor (attenuation), hypersensibility, paresthesia (sensory falsification). In addition, it may be grouped into hemi-sensory disturbance, superficial sensory disturbance, systemic sensory disorder, depending on the site with sensory disturbances.

"Survival rate improvement" means that, when the compound for use in the invention is administered to mammals already having an cerebral stroke attack, then the survival rate of the mammals is significantly increased as compared with that of non-administered mammals.
Preferably, it means that, when the compound is administered to humans, then the survival rate thereof for from 1 month to 12 months, for from 1 year to 5 years, or for 5 years or more becomes longer than that of other human cases that have underwent any other various therapeutical treatment for the disorder or that have not underwent it.

"After onset of the stroke" is meant to indicate cases already having had an cerebral stroke attack before administration of a test substance thereto. In particular, it is preferably after onset of the primary cerebral stroke attack. The presence or absence of cerebral stroke attack is judged by body weight change and/or neurologic symptom observation. During the observation period, when abnormal body weight change and/or neurologic symptom observation is found, and preferably when abnormal body weight change and neurologic symptom observation is found, then it is recognized that the case has had an cerebral stroke attack.

"Abnormal body weight change" means no body weight increase or means body weight reduction. "Body weight reduction" means that the body weight reduces within 24 hours, and is preferably a rapid body weight reduction is employed as an index for it. "Neurologic symptom" has the same meaning as above, concretely including motor paralysis and ataxia.

"Stroke-prone spontaneously hypertensive rat" is those having a mean blood pressure of 187 mmHg when they are 10 weeks old, and having an cerebral stroke attack (both brain hemorrhage and brain infarction) that is pathologically extremely similar to that of humans. Up to the present, there are known four lines of SHRSP/Hos (by Hoshino Experimental Animal Breeder, Nippon SLC), SHRSP/Izm (by Shimizu Experimental Material, Funabashi Farm, Nippon SLC), SHRSP/NIco (by Charles River), and SHRSP/Sea (by Seac Yoshitomi), and they are all available from the indicated organs. Preferred are those of SHRSP/Izm.

"Scoring" means that the degree of body weight change or the degree of neurologic symptom of test cases is scored.

Regarding the body weight change, body weight increase is considered normal state, and no body weight change as well as body weight reduction is considered as cerebral stroke attack occurrence, and its degree is scored. Concretely, the body weight reduction of from 0 to 5 g is scored as light cerebral stroke recurrence (score 1); the body weight reduction of 20 g or more is scored as heavy cerebral stroke recurrence (score 3); and the body weight reduction of from 5 g to less than 20 g is scored as middle cerebral stroke recurrence (score 2). The total of the scores of each individual for 7 days after cerebral stroke attack occurrence is the final score of the week; and the maximum score is 21. Depending on the ambient condition, the observation period may be suitably changed for the evaluation.

Regarding the neurologic symptom, both the fore-legs and both the hind legs of a test case are checked for the paralysis and the activity depression as the items of the neurologic symptoms thereof, and they are scored depending on the degree of the symptoms. Concretely, according to a reference (European Journal of Pharmacology, 192, 165-167, 1991), they are normal state (score 0), light symptom (score 1), middle symptom (score 2) and heavy symptom (score 3); and the final data of an individual are the total of the scores of these items. The maximum score is 15.

Not specifically defined, "test substance" includes, for example, novel synthetic compounds, commercially-available compounds (including peptides), various known compounds (including peptides) registered in chemical files, compound groups obtained by combinatorial chemistry technology (N. K. Terrett. M. Gardner, D. W. Gordon, R. J. Kobylecki, J. Steele, Tetrahedron, 51, 8135-73 (1995)), cultured microorganisms supernatants, natural components derived from plants and marine organisms, and animal tissue extracts.

The compound for use in the invention may be formulated into various oral or parenteral administration forms of a broad range, and may be administered. Specifically, the compound for use in the invention may be administered intravenously, intramuscularly, intracutaneously, subcutaneously, intraduodenally or intraabdominally by injection. In addition, the compound for use in the invention may be administered by inhalation, for example, intranasally. Further, the invention for use in the invention may be administered percutaneously. As will be obvious to those skilled in the art, the administration modes mentioned below may include the compound for use in the invention or its corresponding pharmaceutically-acceptable salt, as an active ingredient.

When the compound for use in the invention is formulated into a pharmaceutical composition containing it, the pharmaceutically-acceptable carrier for it may be solid or liquid.

The solid-form preparations include powders, tablets, pills, capsules, cachets, suppositories and dispersive granules. The solid carrier may be one or more substances capable of serving as diluent, flavoring, solubilizer, lubricant, suspending agent, binder, antiseptic, tablet disintegrator or inclusion substance. In the powder, the carrier may be a fine solid capable of being mixed with a fine active ingredient. For the tablet, the active ingredient is mixed with a binder carrier in a suitable ratio, and tabletted into a desired form having a desired size.

The powder and the tablet preferably contain about 2%, or from 10% to about 70% of an active compound. Suitable carriers for them are magnesium carbonate, magnesium stearate, talc, sugar, lactose, pectin, dextrin, starch, gelatin, tragacanth gum, methyl cellulose, sodium carboxymethyl cellulose, low-melting-point wax, cocoa butter.

The preparation is meant to indicate a preparation form that contains an inclusion substance as a carrier and an active compound, including, for example, those in which the active ingredient is surrounded by a carrier and which may contain or may not contain any other carrier, or those in which the active ingredient is encapsulated along with the carrier. Similarly, it may include cachets and lozenges. Those tablets, powders, capsules, pills, cachets and lozenges may be used as solid administration forms suitable to oral administration.

In producing suppositories, a low-melting-point wax, for example, a mixture of fatty acid glycerides or cocoa butter is first melted, and an active ingredient is uniformly dispersed into it, for example, by stirring. Then, the thus-melted homogeneous mixture is cast into a mold having a suitable size, cooled and solidified. The liquid preparation includes solution, suspension, retentive enema and emulsion, for example, water or aqueous propylene glycol solution.

For parenteral injection, the liquid preparation may be formulated as an aqueous polyethylene glycol solution. The aqueous solution suitable for oral administration may be produced by dissolving an active ingredient in water and optionally adding suitable colorant, flavoring, stabilizer and thickener thereto. The aqueous suspension suitable for oral administration may be produced by dispersing a fine active ingredient in water along with a viscous substance, for example, natural or synthetic rubber, resin, methyl cellulose, sodium carboxymethyl cellulose and any other known suspending agent therein.

The invention also encompasses a solid form preparation that is intended to be converted into a liquid preparation for oral administration just before use. The liquid preparation form of the type includes solution, suspension and emulsion. These preparations may contain, in addition to the active ingredient therein, any of colorant, flavoring, stabilizer, buffer, artificial and natural sweetener, dispersant, thickener, solubilizer.

The medical preparation is preferably in the form of a unit dose. For these forms, the preparation is divided into unit dose portions each containing a suitable amount of the active ingredient therein. The unit dose form may be for packaged preparations containing a separated amount of each preparation, for example, as packaged tablets, capsules, or as powder in vials or ampoules. The unit dose form may also be for capsules, tablets, cachets or lozenges themselves, and it may be for packaged preparations that contain any suitable number of them packaged therein. The amount of the active ingredient in the unit dose preparation may be varied or controlled to be from 0.5 to 100 mg, preferably from 2.5 to 80 mg, depending on specific applications or on the titer value of the active ingredient.

Atorvastatin utilized for the application of the invention may be repeatedly administered at an initial dose of from about 2.5 mg/day to about 100 gm/day. Preferably, the dose range is from about 10 mg/day to about 80 mg/day. However, the dose may be varied depending on the necessary condition of the patient, the degree of the disorder to be treated, and the compound to be used. The suitable dose for a specific environment may be determined within a range of the skill of those skilled in the art. In general, the treatment is started from a lower dose than the suitable dose of the compound. Then, the dose may be gradually increased up to that for the optimum effect thereof under the ambient circumstance. For convenience sake and if desired, the overall dose a day may be divided into plural portions and they may be separately administered in one day.

### EXAMPLE

The invention is described in more detail with reference to the following Examples, to which, however, the invention should not be limited.

The effect and the evaluation method of the invention are confirmed according to the test methods mentioned below.

### Example 1:

### (1) Test Substance:

Atorvastatin calcium hydrate (by Pfizer Inc.), a type of atorvastatin, was suspended in 0.5% methyl cellulose, and administered at a dose of 5 ml/kg. The dose was 3 and 30 mg/kg, and it was repeatedly orally administered once a day. The dose in this test was determined with reference to the following publications (Namura S. et al., Pretreatment with atorvastatin protects brain against permanent focal ischemia in mice, 60th Annual Meeting of the Japan Neurosurgical Society, Okayama, Japan, Oct. 24-26, 2001; Sasamata M. et al., The synthetic 3-hydroxy-3-methylglutaryl coenzyme A reductase inhibitor atorvastatin protects brain against permanent focal ischemia in mice, 75th Annual Meeting of the Japan Neurosurgical Society, Kumamoto, Japan, March 13-15, 2002).

### (2) Analysis of Recurrence Rate, Neurologic Symptom and Survival Rate:

SHR-SP male rats (9-week age when bought, by Nippon SLC) were bred under a constant-temperature and constant-humidity condition. The feed was solid SP feed for breeding SHR-SP rats; and every rat was kept to freely drink tap water as automatically supplied thereto. The body weight of each rat was measured everyday, and the rats were checked for an cerebral stroke attack (Stroke, Vol. 20(9), pp. 1212-1218, 1989; Journal of Pharmacology & Experimental Therapeutics, Vol. 291(2), pp. 569-575, 1999). The cerebral stroke attack in the rats of this line is accompanied by neurologic symptoms such as paralysis or hemiplegia (European Journal of Pharmacology, Vol. 192 (1), pp. 165-167, 1991), and abnormal body weight change such as rapid body weight reduction. The abnormal body weight change may be considered to be caused by the deterred drinking and eating owing to the cerebral stroke attack (Brain Research, Vol. 452 (1-2), pp. 323-328, 1988; Stroke, Vol. 20(8), pp. 1089-1091, 1989; European Journal of Pharmacology, Vol. 192 (1), pp. 165-167, 1991). Accordingly, the date on which these neurologic symptoms or abnormal body weight change were confirmed for the first time is the date on which the rats had a primary cerebral stroke attack.

### 1) Cerebral stroke Recurrence Inhibitory Effect:

For scoring the recurrence of cerebral stroke, the weight of each rat with cerebral stroke was measured every day for 14 days from the date of the primary cerebral stroke attack. Since the cerebral stroke attack is accompanied by abnormal body weight change (Brain Research, Vol. 452 (1-2), pp. 323-328, 1988; Stroke, Vol. 20(8), pp. 1089-1091, 1989; European Journal of Pharmacology, Vol. 192 (1), pp. 165-167, 1991), the recurrence of cerebral stroke was scored by the everyday body weight change. The body weight change was measured at a unit of 5 g; and it was scored as follows: Score 0, good with no abnormality (body weight increase); score 1, light abnormality (body weight reduction of at most 5 g); score 2, middle abnormality (body weight reduction of from 10 to 15 g); score 3, heavy abnormality (body weight reduction of 20 g or more). The total of the scores for 7 days after the date of the primary attack of each individual is the final score of that week, and the maximum score is 21.

### 2) Cerebral stroke Recurrence-Accompanied Neurologic Symptom-Improving Effect:

The rats were checked for their neurologic symptoms on the day the first stroke occurred and 7 and 14 days later by the modified method of Yamamoto (Brain Research, Vol. 452(1-2), pp. 323-328, 1988; Stroke, Vol. 20(8), pp. 1089-1091, 1989; European Journal of Pharmacology, Vol. 192(1), pp. 165-167, 1991). Both the fore-legs and both the hind legs of the rats were checked for the paralysis and the activity depression as the items of the neurologic symptoms thereof. These were scored depending on the degree of the symptoms, according to a reference (European Journal of Pharmacology, 192, 165-167, 1991) as follows:
Score 0, good with no abnormality; score 1, light abnormality; score 2, middle abnormality; score 3, heavy abnormality. The final data of the individual rat are the total of the scores of these items. The maximum score is 15.

### 3) The survival rate after onset of the stroke:

After the first stroke, rats in the present study were randomly assigned to one of three groups, namely vehicle (control) or atorvastatin at 3 or 30 mg/kg group (n=33 for each vehicle-treated group and atorvastatin-treated group). Vehicle or atorvastatin was orally administered everyday, once a day from day of the first stroke to death.

### (3) Statistics:

A Steel test was used to the neurologic symptom and recurrence scores, and a Log-rank test was applied to the survival rate. In these, p value of smaller than 0.05 is considerd as a significant difference.

### (4) Result:

Of 99 SHR-SP rats tested, 11 (11.1%) died before the start of administration; and 7 (7.0%) did not have an cerebral stroke attack within 14 weeks after their birth. Therefore, these rats were not used in this test. The remaining rats that had had an cerebral stroke attack within a period of 10 weeks to 13 weeks after their birth (81.8%) were used in the test.

Within 2 weeks after the primary cerebral stroke attack, the test animals of the administration groups gave no significant difference from those of the control group, in point of the diastolic pressure, the systolic pressure, the mean blood pressure, the heart beat, and the total cholesterol level.

### 1) Cerebral stroke Recurrence Inhibitory Effect:

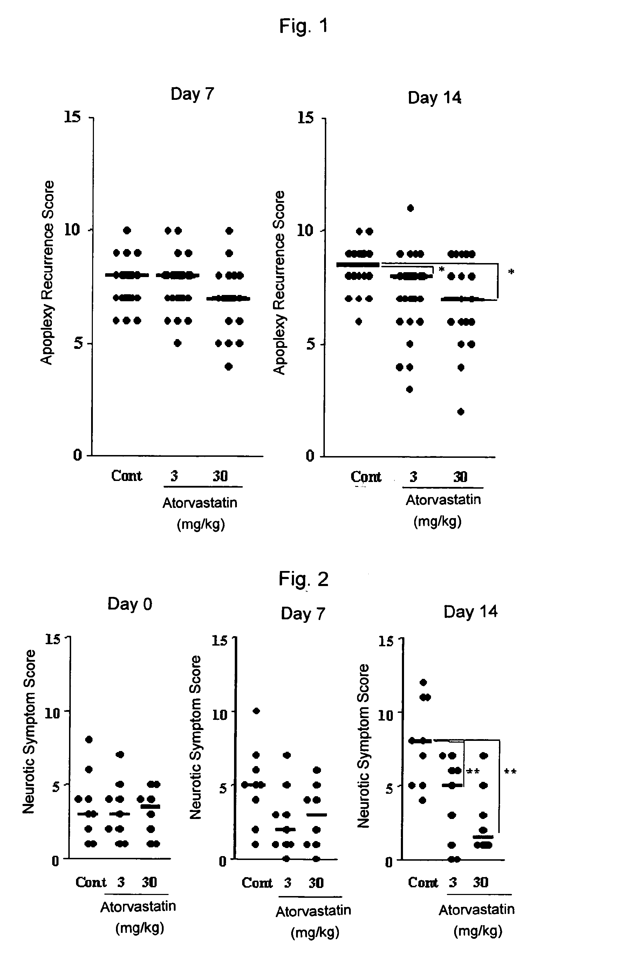

After the primary cerebral stroke attack, the body weight of the rats not showing cerebral stroke recurrence increased, but within 7 days or within 14 days, some rats showed worsening or recurrence of cerebral stroke symptoms, such as abnormal body weight change, paralysis of all legs, activity reduction, aggressiveness increase. The rats of the control group showed significant worsening of symptoms by cerebral stroke recurrence within 7 days and within 14 days after the primary cerebral stroke attack. The rats of the 30 mg/kg/day administration group showed improved symptoms on day 7 after the primary cerebral stroke attack; and on day 14 after it, the rats of both the 3 mg/kg/day administration group and the 30 mg/kg/day administration group showed a significant improvement (Fig. 2).

These results confirm that atorvastatin is effective for preventing cerebral stroke recurrence.

### 2) Cerebral stroke Recurrence-Accompanied Neurologic Symptom-Improving Effect:

The apoplectic rats showed abnormal body weight change, as well as paralysis of all legs, activity reduction or aggressiveness increase. On day 14 after the primary cerebral stroke attack, the neurologic symptoms of the rats of the control group significantly worsened as compared with those on the date of the primary cerebral stroke attack. On day 7 after the primary cerebral stroke attack, the neurologic symptoms of the rats of the atorvastatin (3 and 30 mg/kg/day, p.o.) administration groups were improved (Fig. 1). In both groups, the rats showed a significant improvement on day 14 after the primary cerebral stroke attack; and the neurologic symptoms of the rats in the 30 mg/kg administration group were restored nearly to the ordinary level.

These results confirm that atorvastatin is effective for improving cerebral stroke recurrence-accompanied neurologic symptoms.

### 3) The Survival Rate after onset of the stroke:

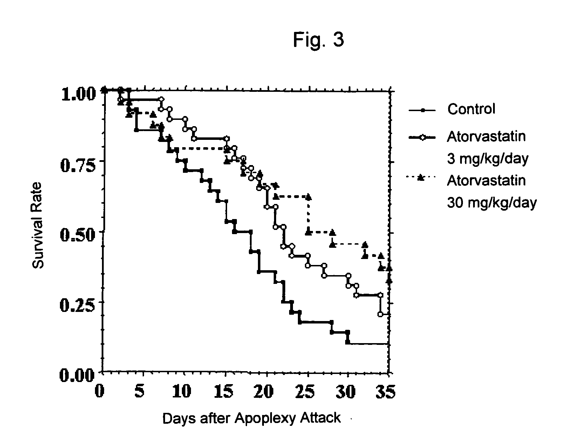

On day 35 after the primary cerebral stroke attack, the survival rate of the rats of the control group was 3/28 (10.7%). The survival rate of the rats of the atorvastatin 3 and 30 mg/kg/day administration groups increased dose-dependently; and their survival rate was 6/29 (20.7%) and 8/24 (33.3%), respectively (Fig. 3). The improvement by 30 mg/kg/day administration showed a significant difference in the Log-rank test.

These results confirm that atorvastatin is effective for improving survival rate after onset of the stroke.

From the results of the neurologic symptom-improving effect and the survival rate-improving effect, it was also confirmed that atorvastatin has an cerebral stroke recurrence inhibitory effect.

Similarly, according to the above-mentioned evaluation methods, pitavastatin and rosuvastatin may be confirmed for the cerebral stroke recurrence inhibitory effect, the cerebral stroke recurrence-accompanied neurologic symptom-improving effect and the survival rate after onset of the stroke-improving effect thereof.

Further, it has been confirmed that the evaluation method for cerebral stroke recurrence inhibitory effect of the invention is an excellent method for readily evaluating the presence or absence of cerebral stroke recurrence and the degree thereof, not requiring the brain tissue observation by high-level technology. In addition, it has also been confirmed that the evaluation method for cerebral stroke recurrence-accompanied neurologic symptom-improving effect and survival rate after onset of the stroke-improving effect is also a simple and useful method that utilizes the index capable of readily judging the presence or absence of cerebral stroke recurrence.

### INDUSTRIAL APPLICABILITY

Using stroke-prone spontaneously hypertensive (SHR-SP) rats, the invention has confirmed the effectiveness of atorvastatin for preventing cerebral stroke recurrence, for relieving cerebral stroke recurrence-accompanied neurologic symptoms, and for improving survival rate after onset of the stroke, and atorvastatin is therefore useful as an cerebral stroke recurrence inhibitor, an cerebral stroke recurrence-accompanied neurologic symptoms reliever, and a survival rate after onset of the stroke improver.

In addition, the evaluation method of the invention is useful as a simple method for evaluating the cerebral stroke recurrence inhibitory effect, the neurologic symptom relieving effect and the survival rate improving effect of a compound.

## Claims

1. An cerebral stroke recurrence inhibitor comprising atorvastatin or a pharmaceutically acceptable salt thereof as an active ingredient.

2. The cerebral stroke recurrence inhibitor as claimed in claim 1, which is for a mammal not having ischemic heart disease as a underlying disease thereof.

3. The cerebral stroke recurrence inhibitor as claimed in claim 1 or 2, which is for a hypertensive mammal.

4. An cerebral stroke recurrence-accompanied neurologic symptom reliever comprising atorvastatin or a pharmaceutically acceptable salt thereof as an active ingredient.

5. A survival rate after onset of the stroke improver comprising atorvastatin or a pharmaceutically acceptable salt thereof as an active ingredient.

6. A method of evaluating the cerebral stroke recurrence inhibitory effect of a test substance, the cerebral stroke recurrence-accompanied neurologic symptom-relieving effect thereof or the survival rate after onset of the stroke-improving effect thereof, which comprises the following steps:
(a) observing the body weight change and/or the neurologic symptom of a stroke-prone spontaneously hypertensive rat before administration of a test substance thereto,
(b) selecting the rat that has shown abnormal body weight change and/or neurologic symptoms during the observation in step (a),
(c) administering a test substance to the rat selected in has (b) and observing them for a predetermined period of time.

7. A method of evaluating the cerebral stroke recurrence inhibitory effect of a test substance, which comprises administering a test substance to a stroke-prone spontaneously hypertensive rat that has had an cerebral stroke attack, followed by checking the rat for the body weight change and/or the neurologic symptom change thereof for the index of the effect of the test substance.

8. A method of evaluating the cerebral stroke recurrence inhibitory effect of a test substance, which comprises administering a test substance to a stroke-prone spontaneously hypertensive rat that has had an cerebral stroke attack, followed by scoring the degree of the body weight change of the rat.

9. A method of evaluating the cerebral stroke recurrence-accompanied neurologic symptom-relieving effect of a test substance, which comprises administering a test substance to a stroke-prone spontaneously hypertensive rat that has had an cerebral stroke attack.

10. A method of evaluating the cerebral stroke recurrence-accompanied neurologic symptom-relieving effect of a test substance, which comprises administering a test substance to a stroke-prone spontaneously hypertensive rat that has had an cerebral stroke attack, followed by scoring the degree of the neurologic symptom of the rat.

11. A method of evaluating the survival rate after onset of the stroke-improving effect of a test substance, which comprises administering a test substance to a stroke-prone spontaneously hypertensive rat that has had an cerebral stroke attack.
